# EUROPEAN PATENT SPECIFICATION

(11) **EP 3 745 413 B1**
(45) Date of publication and mention of the grant of the patent: **04.10.2023**
(21) Application number: 20177628.3
(22) Date of filing: 30.05.2020
(51) Int. Cl.: G16H 50/20, G16H 50/70, G16H 30/40, G16H 30/20, G16H 50/30

(54) **A METHOD AND SYSTEM FOR PREDICTING NEUROLOGICAL DISEASE**
VERFAHREN UND SYSTEM ZUR VORHERSAGE EINER NEUROLOGISCHEN KRANKHEIT
PROCÉDÉ ET SYSTÈME POUR PRÉDIRE UNE MALADIE NEUROLOGIQUE

(30) Priority: 01.06.2019 EP 19177790
(43) Date of publication of application: 02.12.2020
(73) Proprietor: Inteneural Networks Inc., Chicago, IL 60610 (US)
(72) Inventor: Kraft, Marek, 61-251 Poznan (PL); Lewicki, Paul, Tulsa, Oklahoma 74114 (US); Siemionow, Kris B., Chicago, Illinois 60610 (US); Pieczynski, Dominik, 63-004 Tulce (PL); Mikolajczak, Michal, 61-415 Poznan (PL); Pawlak, Mikolaj Andrzej, 61-614 Poznan (PL); Klimont, Michal, 60-653 Poznan (PL)
(74) Representative: Kancelaria Eupatent.pl Sp. z.o.o

(56) References cited:
- WO-A2-2019/103912
- US-A1- 2011 129 130
- Mina Rezaei ET AL: "A Conditional Adversarial Network for Semantic Segmentation of Brain Tumor" In: "Annual International Conference on the Theory and Applications of Cryptographic Techniques, EUROCRYPT 2018", 17 August 2017 (2017-08-17), Springer, Berlin, Heidelberg 032548, XP055452166, ISBN: 978-3-642-17318-9 vol. 10670, pages 241-252, DOI: 10.1007/978-3-319-75238-9_21, * the whole document *

## Description

### TECHNICAL FIELD

The present invention relates to prediction of a probability that the patient may have a particular neurological disease by utilizing machine learning, voice recognition, and MRI derived brain biomarkers.

### BACKGROUND

Neurodegeneration is a term used to describe a wide range of conditions and diseases which primarily affect the neurons in the human brain. Strictly speaking neurodegeneration refers to the progressive atrophy (death) and loss of function of neurons, which is present in neurodegenerative diseases such as Alzheimer's disease, Huntington's disease, and Parkinson's disease. It is important to note that neurons normally do not regenerate, replace, or reproduce themselves, so when they become damaged or die they cannot be replaced by the body. Neurodegenerative diseases are incurable and debilitating conditions that result in progressive degeneration and / or death of nerve cells. This causes problems with movement (called ataxias), or mental functioning (called dementias). Frequently, both movement and mental symptoms are present in patients suffering from neurodegenerative disorders. Dementias are responsible for the greatest burden of neurodegenerative diseases, with Alzheimer's representing approximately 60-70% of dementia cases. There is a need to be able to increase the time to diagnosis as well as diagnose the above mentioned disorders at an earlier stage in order to increase the efficacy of treatment and improve clinical outcomes.

Current problems associated with treatment of neurodegenerative conditions are a result of limited diagnostic information about a given patient. Physicians rely on history, physical exam, blood work, and brain MRI to arrive at a diagnosis and make treatment recommendations. This is a limited approach as the sensitivity of the analysis is restricted by many factors such as; the ability of the physician to discern visible pathology from the imaging and correlate it with a disease process and stage of disease; ability to identify mild subclinical speech anomalies; ability of the physician to quantify and correlate lab work data with a specific disease process (e.g. CBC, CMP, CRP in Huntington's versus Alzheimer's); and finally the physician's ability to take multiple data points from various diagnostic modalities (e.g. genetic testing, CBC, BMP, CRP, MRI, BMI, speech, etc.) and create a health "portrait" of a given patient. Currently, the physician does not have the ability not only to create such an objective health "portrait", but they can not compare it to a database of many such health "portraits" with known neurological diagnosis and just as importantly know the historical outcomes of treatment for those individuals. Key MRI components of the health "portrait" of an individual with neurodegenerative process are brain age, brain health, and the gyrification coefficient. Brain age, defined as the difference between the estimated age and the biological age of the individual, has been suggested to be a reliable, MRI scanner-independent, and efficient measure of deviation from normal (statistically speaking) brain aging in healthy participants and to be able to predict individual brain maturation. Different research groups found brain age to be correlated with physical fitness, mortality risk in elderly participants, and human immunodeficiency virus status, and cognitive performance. Others have shown that, compared with control groups, brain age was higher in patients with psychiatric disorders, mild cognitive impairment, Alzheimer's disease, or diabetes and much lower for long-term meditators. Taken together, these findings provide strong evidence that age can be estimated using features from structural brain imaging and can be meaningfully related to other age-related processes. Brain health is based on an evaluation of the combined effects of whole brain tissue atrophy (brain wasting secondary to nerve cell death) and vascular disease in a single measure. About 15% of the freshly oxygenated blood pumped out by the heart goes to the brain. The brain itself has a very robust network of blood vessels and capillaries. These vessels are prone to disease as a result of high blood pressure, aging, high cholesterol, diabetes and other disorders. Presence of small vessel disease and brain tissue atrophy (death) both increase with age, are often present together, and are risk factors for stroke, dementia, and neurodegeneration. The importance of vascular disease on accelerating neurodegenerative pathologies and cognitive decline has recently been recognized. Moreover, structural changes in the brain are rarely (if at all) monitored in a continuous way. Using longitudinal data enables tracking of long-term changes in the patient's brain and makes diagnosis more accurate.

There are known several attempts to develop computer systems to provide treatment recommendations.

For example, a PCT application WO2017210502A1 discloses a system for providing a treatment recommendation for a patient having a depression disorder. The system comprises a server computer configured to receive patient information including patient responses to each of a plurality of questions provided on a questionnaire; process, with a trained statistical model, a set of values determined based, at least in part, on at least some of the received patient information to determine treatment recommendation information for the patient; and transmit the treatment recommendation information to an electronic device.

A US patent application US20120016206A1 discloses a system and method of recommending a treatment among a plurality of treatment options for a given medical condition of a patient. The system receives patient information related to the patient and the medical condition, and searches, at least in part in a computer process, a database with a plurality of indexed studies relating to the plurality of different treatment options for the given medical condition. The system then assigns a study value to each of the plurality of studies, and determine the applicability of the studies to the patient using the patient information to produce a plurality of applicability values. At least the study values and the applicability values are used to generate treatment scores for the treatment options for generating a report listing the treatment options and a) the treatment scores and/or b) information derived from the treatment scores.

A US patent application US20140303986A1 discloses a method of determining an optimal treatment, the method comprising determining a frequency for each health care provider indicating how frequently each treats a selected disease; determining for each health care provider, their average patient outcome APO for treating the selected disease; determining a score for each health care provider based on the corresponding frequency and APO; determining which of the health care providers are experts from the scores that exceed a predefined threshold; and selecting a treatment prescribed by at least one of the identified experts as the optimal treatment.

A PCT application WO2019103912A2 discloses a machine learning system to process data relating to potentially cancerous lung and liver lesions.

A publication by Rezaei, Mina, et al. "A conditional adversarial network for semantic segmentation of brain tumor" (International MICCAI Brainlesion Workshop. Springer, Cham, 2017) discloses automated brain lesion detection by a network including fully convolutional neural networks.

US2011/129130A1 discloses the determination of a patient disease severity score based on patient image data and patient non-image data, said severity score being indicative of a neurodegenerative condition of the patient.

There is a need to further develop systems for predicting the probability that a subject may have a particular neurological disease.

### SUMMARY OF THE INVENTION

The present invention relates to a computer-implemented method and system for predicting a probability that a patient may have a particular disease according to the appended claims.

### BRIEF DESCRIPTION OF DRAWINGS

The present invention is shown by means of exemplary embodiments on a drawing, wherein:
Fig. 1 shows an overview of the system;
Fig. 2 shows a brain age determining convolutional neural network (CNN) architecture;
Fig. 3 shows a computer-implemented system.

### DETAILED DESCRIPTION

The computer system and method as presented herein is intended to augment the work of a physician and is not intended to replace the doctor. The system, and in particular its scoring module 130, analyzes multiple data points derived from diagnostic tests and creates a health "portrait" of an individual suspected of having a neurodegenerative condition. This "portrait" is then compared to a large data set composed of normal and pathological "portraits" of individuals with neurodegenerative conditions. With a large amount of cases for unremarkable (healthy) brains and brains with symptoms of a range of conditions, the system is able to place the patient on a spectrum for a wide range of indicators signaling the presence and in some cases also the severity of the condition of the patient. The goal of the computer system is to provide and objective datapoint that would augment the physician's decision making and diagnostic capabilities, which are based on traditional approaches of medical practice (e.g. history and physical).

Therefore, the system can provide:
- objective, automated analysis with specific, interpretable measurements comparable across patients;
- capability to perform the comparative analysis with a vast database of scans of both healthy and problematic cases in separate age groups;
- the capability to perform a trend analysis - growing/shrinking/changes of structures, tumors, pathologies etc.;
- time savings, as the process is automated;
- multimodal analysis (multiple sequences at the same time, registered) in the case of some functionalities (brain health), imaging + voice/speech analysis.

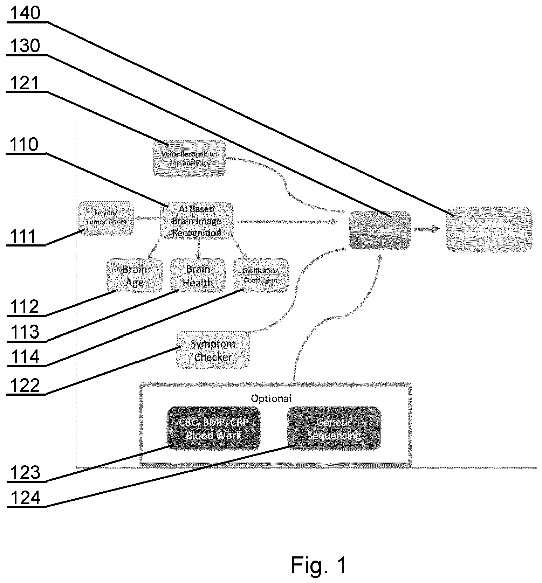

The system of the present invention is shown in an overview on Fig. 1. It comprises the following modules.

An Al-based (artificial intelligence) brain image recognition module 110 is configured to process brain image data to determine various parameters related to the brain. It comprises the following sub-modules. A lesion/tumor check sub-module 111 is configured to check whether the brain image is indicative of any tumors or lesions, and if so, what is their number and volume. A brain age sub-module 112 is configured to compute a brain age, and in addition it may provide information on which areas of the brain contributed to decision related to the particular brain age (therefore, it may indicate where the potential cause of problem is located). A brain health sub-module 113 is configured to determine potentially healthy and unhealthy brain areas, such as by quantifying visible brain injury from small vessel disease and brain atrophy. A gyrification coefficient module 114 is configured to determine a gyrification index that indicates the amount of folding of the surface of the brain, which can be provided as an overall (global) gyrification index and/or a local gyrification index (cortical folding) (relating to particular areas of the brain surface) (the role of gyrification index is described e.g. in a publication by Jonathan MHarris et al. "Abnormal cortical folding in high-risk individuals: a predictor of the development of schizophrenia?" (Biological Psychiatry, Volume 56, Issue 3, 1 August 2004, Pages 182-189)).

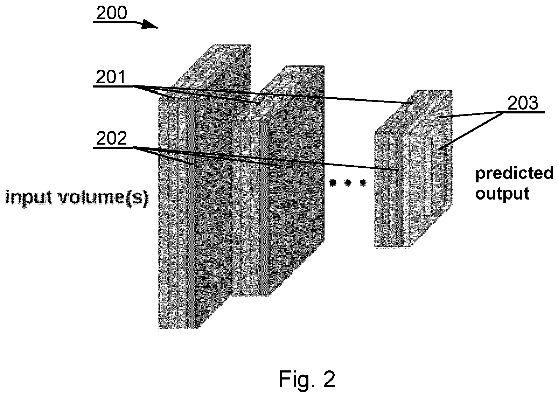

The brain image recognition module 110 and its sub-modules 111-114 may be implemented by means of at least one Convolutional Neural Network (CNN) 200, such as shown in Fig. 2. The network has a contracting path comprising 3D convolutional layers 201, pooling layers 202 and dense layers 203. Each convolutional layer 202 has a plurality of filters (for example, from 16 to 512 filters). Convolutions may be of the regular kind or dilated convolutions. A different stride of Sc (for example: 1, 2, 4 or 8) can be set for each convolutional layer 201. The 3D maximum pooling layers 202 may have optional added dropout or other regularization. A different stride of S_{MP} (for example, 1, 2, 4 or 8) can be set for each pooling layer 202. The dense layers 203 may have an optionally added dropout or other regularization.

The network is configured to accept as a primary input one or more medical images (preferably, 3D volumes) of the brain to be analyzed. For example, different image types of the brain can be provided, such as T1 and T2-weighted volumes, or images made with and without contrast. Once the network is trained for the specific task, it can provide, as output, a parameter of the particular sub-module 111-114 as discussed above.

The system further comprises the following additional modules.

A voice recognition and analytics module 121 may analyze the patient's capability to retell a story that the patient heard/seen on audio/video or read as a text. This can be a strong indicator od Alzheimer's. The module can operate as explained e.g. in a publication by Juciclara Rinaldi et al, "Textual reading comprehension and naming in Alzheimer's disease patients" (Dement Neuropsychol. 2008 Apr-Jun; 2(2): 131-138). The output can be provided e.g. as a value indicating the capabilities of the patient, e.g. on a scale from 1 to 10.

A symptom checker module 122 is configured to determine additional symptoms such as blurry vision, speech disorders, hypertension, memory problems, headaches. This can be done by simply asking the patient a series of simple yes/no questions related to particular symptoms. The output data is used as additional input to the machine learning algorithms and makes diagnoses and predictions more accurate.

The blood work module 123 may be used to input to the system values of results of blood tests, such as CBC, BMP, CRP.

The genetic sequencing module 124 may be used to input to the system DNA data, which may be indicative of heritable neurological disorders.

The scoring module 130 operates as described in the initial section of this detailed description, to determine a score related to a probability that the patient may have a particular disease, based on the inputs from the other modules 110, 121-124. As a result, by using more input signals for prediction, results in increased accuracy. The system may make a decision based not only on the current data, but also on historical data: combining multi-domain data to make a final diagnosis and tracking long-time changes of crucial coefficients and marker levels is therefore very beneficial.

Finally, a treatment recommendation module 140 recommends predicted treatment.

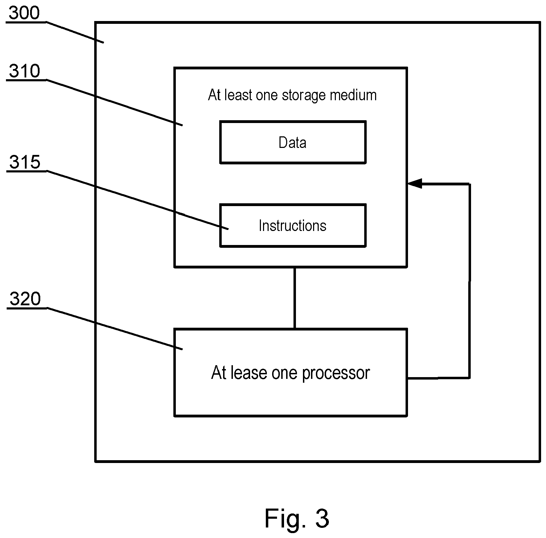

The functionality of the system of Fig. 1 can be implemented in a computer-implemented system 300, such as shown in Fig. 3. The system may include at least one non-transitory processor-readable storage medium that stores at least one of processor-executable instructions or data and at least one processor communicably coupled to at least one non-transitory processor-readable storage medium. At least one processor is configured to perform the steps of the methods presented herein. The computer-implemented system 300, for example a machine-learning system, may include at least one non-transitory processor-readable storage medium 310 that stores at least one of processor-executable instructions 315 or data; and at least one processor 320 communicably coupled to the at least one non-transitory processor-readable storage medium 310. At least one processor 320 may be configured to (by executing the instructions 315) to perform the functionality of the modules of Fig. 1.

Although the invention is presented in the drawings and the description and in relation to its preferred embodiments, these embodiments do not restrict nor limit the presented invention. It is therefore evident that changes, which come within the meaning and range of equivalency of the essence of the invention, may be made. The presented embodiments are therefore to be considered in all aspects as illustrative and not restrictive. According to the abovementioned, the scope of the invention is not restricted to the presented embodiments but is indicated by the appended claims.

## Claims

1. A computer-implemented method for predicting a probability that a patient may have a particular neurological disease , the method comprising:
- analyzing a pre-stored brain image of the patient by means of a Convolutional Neural Network, CNN, (110, 200) to determine brain image analysis results including a presence of a tumor or lesion (111), brain age (112), brain health (113) based on an evaluation of the combined effects of whole brain tissue atrophy and vascular disease in a single measure, and gyrification coefficient (114):
- receiving additional data, including: voice recognition index (121) corresponding to the patient's capability to retell a story and additional symptom checks (122) corresponding to at least one of blurry vision, speech disorders, hypertension, memory problems or headaches,
- combining (130) the brain image analysis result with the additional data (121-124) to create a health portrait of the patient and comparing said health portrait with a dataset composed of normal and pathological portraits of other individuals with neurodegenerative conditions to determine a score related to the probability that the patient may have a particular neurological disease.

2. The method according to claim 1, wherein the additional data further includes at least one of: blood work results (123) corresponding to values of results of blood tests; or genetic sequencing results (124) corresponding to DNA data.

3. The method according to claim 1 or 2, further comprising recommending (140) a neurological treatment for the patient by based on the score.

4. A computer-implemented system for performing the method of any of claims 1 to 3.

## Patentansprüche

1. Computerimplementiertes Verfahren zur Vorhersage einer Wahrscheinlichkeit, dass ein Patient eine bestimmte neurologische Krankheit haben könnte, wobei das Verfahren Folgendes umfasst:
- Analysieren eines vorgespeicherten Gehirnbildes des Patienten mittels eines neuronalen Faltungsnetzwerks, CNN, (110, 200), um Gehirnbildanalyseergebnisse zu bestimmen, beinhaltend ein Vorhandensein eines Tumors oder einer Läsion (111), Gehirnalter (112), Gehirngesundheit (113) basierend auf einer Bewertung der kombinierten Auswirkungen von Atrophie des gesamten Gehirngewebes und Gefäßkrankheit in einer einzelnen Messung, und Gyrifizierungskoeffizient (114);
- Empfangen von zusätzlichen Daten, beinhaltend: Spracherkennungsindex (121), welcher der Fähigkeit des Patienten entspricht, eine Geschichte nachzuerzählen, und zusätzliche Symptomprüfungen (122), die mindestens einem von verschwommenem Sehen, Sprachstörungen, Bluthochdruck, Gedächtnisproblemen oder Kopfschmerzen entsprechen,
- Kombinieren (130) des Gehirnbildanalyseergebnissen mit den zusätzlichen Daten (121-124), um ein Gesundheitsporträt des Patienten zu erstellen und Vergleichen des Gesundheitsporträts mit einem Datensatz, der aus normalen und pathologischen Porträts anderer Individuen mit neurodegenerativen Zuständen besteht, um einen Wert zu bestimmen, der sich auf die Wahrscheinlichkeit bezieht, dass der Patient eine bestimmte neurologische Krankheit haben könnte.

2. Verfahren nach Anspruch 1, wobei die zusätzlichen Daten ferner mindestens eines von Folgendem beinhalten: Blutuntersuchungsergebnissen (123), die Werten von Ergebnissen von Blutuntersuchungen entsprechen; oder genetischen Sequenzierungsergebnissen (124), die DNA-Daten entsprechen.

3. Verfahren nach Anspruch 1 oder 2, ferner umfassend Empfehlen (140) einer neurologischen Behandlung für den Patienten durch basierend auf der Bewertung.

4. Computerimplementiertes System zur Durchführung des Verfahrens nach einem der Ansprüche 1 bis 3.

## Revendications

1. Procédé implémenté par ordinateur permettant de prédire une probabilité qu'un patient puisse être atteint d'une maladie neurologique particulière, le procédé comprenant :
- l'analyse d'une image cérébrale préenregistrée du patient au moyen d'un réseau neuronal convolutif, CNN, (110, 200) pour déterminer les résultats d'analyse d'image cérébrale, comprenant une présence d'une tumeur ou d'une lésion (111), l'âge du cerveau (112), la santé cérébrale (113) sur la base d'une évaluation des effets combinés de l'atrophie du tissu cérébral total et d'une maladie vasculaire en une seule mesure, et le coefficient de gyrification (114) ;
- la réception de données supplémentaires, comprenant : l'indice de reconnaissance vocale (121) correspondant à la capacité du patient à raconter à nouveau une histoire et des contrôles de symptômes supplémentaires (122) correspondant à au moins un symptôme parmi une vision floue, des troubles de la parole, l'hypertension, des problèmes de mémoire ou des maux de tête,
- la combinaison (130) du résultat d'analyse d'image cérébrale aux données supplémentaires (121 à 124) pour créer un portrait de santé du patient et comparer ledit portrait de santé à un ensemble de données composé de portraits normaux et pathologiques d'autres individus atteints d'états neurodégénératifs pour déterminer un score lié à la probabilité que le patient puisse être atteint d'une maladie neurologique particulière.

2. Procédé selon la revendication 1, lesdites données supplémentaires comprenant en outre au moins un résultat parmi : des résultats d'analyse de sang (123) correspondant à des valeurs de résultats de tests sanguins ; ou des résultats de séquençage génétique (124) correspondant à des données d'ADN.

3. Procédé selon la revendication 1 ou 2, comprenant en outre la recommandation (140) d'un traitement neurologique pour le patient en se basant sur le score.

4. Système implémenté par ordinateur permettant d'exécuter le procédé selon l'une quelconque des revendications 1 à 3.
